# EUROPEAN PATENT SPECIFICATION

(11) **EP 3 505 184 B1**
(45) Date of publication and mention of the grant of the patent: **30.12.2020**
(21) Application number: 18824327.3
(22) Date of filing: 29.06.2018
(51) Int. Cl.: A61K 45/00, A61K 31/216, A61K 31/5375, A61P 35/00, A61P 35/04, A61P 43/00

(54) **INHIBITOR OF POSTOPERATIVE CANCER RECURRENCE AND/OR METASTASIS**
INHIBITOR VON POSTOPERATIVEN REZIDIVEN UND/ODER METASTASEN
INHIBITEURS DE LA RÉCURRENCE ET/OU DE LA MÉTASTASE POSTOPÉRATOIRES DU CANCER

(30) Priority: 30.06.2017 JP 2017128968
(43) Date of publication of application: 03.07.2019
(60) Divisional application: 20206625.4
(73) Proprietor: ONO Pharmaceutical Co., Ltd., Osaka-shi, Osaka 541-8526 (JP); Nippon Medical School Foundation, Tokyo 113-8602 (JP)
(72) Inventor: SAKAMOTO, Atsuhiro, Tokyo 113-8602 (JP); OKAMURA, Tatsuaki, Osaka-shi Osaka 541-8526 (JP); HARADA, Tomohiro, Osaka-shi Osaka 541-8526 (JP)
(74) Representative: J A Kemp LLP
(86) International application number: PCT/JP2018/024782
(87) International publication number: WO 2019/004419

(56) References cited:
- JP-A- 2016 526 049
- US-B2- 9 463 198
- WANG H M ET AL: "Improved survival outcomes with the incidental use of beta-blockers among patients with non-small-cell lung cancer treated with definitive radiation therapy.", ANNALS OF ONCOLOGY : OFFICIAL JOURNAL OF THE EUROPEAN SOCIETY FOR MEDICAL ONCOLOGY MAY 2013, vol. 24, no. 5, May 2013 (2013-05), pages 1312-1319, XP002793428, ISSN: 1569-8041
- AYDINER ADNAN ET AL: "Does beta-blocker therapy improve the survival of patients with metastatic non-small cell lung cancer?", ASIAN PACIFIC JOURNAL OF CANCER PREVENTION : APJCP 2013, vol. 14, no. 10, 2013, pages 6109-6114, XP002793429, ISSN: 2476-762X
- WEBERPALS JANICK ET AL: "Pre- and post-diagnostic beta-blocker use and lung cancer survival: A population-based cohort study", SCIENTIFIC REPORTS, vol. 7, 6 June 2017 (2017-06-06), XP002793430,
- MUSSELMAN REILLY P ET AL: "Association between perioperative beta blocker use and cancer survival following surgical resection", EUROPEAN JOURNAL OF SURGICAL ONCOLOGY, LONDON, GB, vol. 44, no. 8, 17 May 2018 (2018-05-17), pages 1164-1169, XP085429420, ISSN: 0748-7983, DOI: 10.1016/J.EJSO.2018.05.012
- AL-NIAIMI, A. et al.: "The impact of perioperative beta blocker use on patient outcomes after primary cytoreductive surgery in high-grade epithelial ovarian carcinoma", Gynecologic Oncology, vol. 143, 2016, pages 521-525, XP029813705, DOI: doi:10.1016/j.ygyno.2016.09.019
- MIO, YASUMI: "Landiolol and esmolol (ultra-short acting beta1-blocking agent) [1] Action to heart", Masui = The Japanese Journal of Anesthesiology, vol. 55, 2006, pages 841-848, XP009514248, ISSN: 0021-4892
- CATA, JP et al.: "Perioerative beta-blocker use and survival in lung cancer patients", Journal of Clinical Anesthesia, vol. 26, no. 2, 2014, pages 106-117, XP055583974, ISSN: 0952-8180, DOI: 10.1016/j.jclinane.2013.10.004

## Description

### TECHNICAL FIELD

The invention provides a short-acting beta-blocker for use in suppressing recurrence and/or metastasis of non-small cell lung cancer, wherein the short-acting beta-blocker is selected from the group consisting of landiolol, esmolol, and a salt thereof and administered during a perioperative period of cancer surgery. Hereinafter the agent is also referred to as "the agent of the disclosure."

### BACKGROUND ART

Activation of sympathetic nerves has been thought to increase risk of cancer metastasis by promoting angiogenesis and vascular invasion of tumor cells (see Non-Patent Literature 1). Retrospective studies to evaluate effects of oral beta-blockers revealed that chronic administration of a beta-blocker prolonged Overall Survival (OS) or Progression Free Survival (PFS) and improved long-term prognosis in patients with breast cancer, non-small cell lung cancer, prostate cancer, ovarian cancer, and malignant melanoma (see Non-Patent Literatures 2 to 8). A meta-analysis revealed that beta-blockers effectively improved long-term overall survival (see Non-Patent Literature 9). Additionally, there was a clinical trial by a combination of a beta-blocker for oral administration and a COX-2 inhibitor (see Non-Patent Literatures 10 and 11). On the contrary, it was also reported that administration of a beta blocker during a perioperative period did not improve recurrence-free survival rate or overall survival rate in patients with lung cancer (see Non-Patent Literature 12).

Landiolol hydrochloride (trade name: ONOACT), a compound disclosed in Patent Literature 1, has been known as a short-acting selective β₁-blocker and has been primarily approved and marketed as a medicament for modulating heart rate in tachyarrhythmia.

### REFERENCES

### PATENT LITERATURE

[Patent Literature 1] JP3302647B

### NON-PATENT LITERATURE

[Non-Patent Literature 1] Nature Reviews Cancer, Vol. 15, No. 9, pp. 563-572 (2015)
[Non-Patent Literature 2] Journal of Clinical Oncology, Vol. 29, No. 19, pp. 2635-2644 (2011)
[Non-Patent Literature 3] Annals of Oncology, Vol. 24, pp. 1312-1319 (2013)
[Non-Patent Literature 4] Journal of Clinical Oncology, Vol. 29, No. 19, pp. 2645-2652 (2011)
[Non-Patent Literature 5] Oncotarget, Vol. 1, No. 7, pp. 628-638 (2010)
[Non-Patent Literature 6] OncoTargets and Therapy, Vol. 8, pp. 985-990 (2015)
[Non-Patent Literature 7] Gynecologic Oncology, Vol. 127, No. 2, pp. 375-378 (2012)
[Non-Patent Literature 8] Cancer Epidemiology, Biomarkers & Prevention, Vol. 20, No. 10, pp. 2273-2279 (2011)
[Non-Patent Literature 9] Journal of Cancer Research and Clinical Oncology, Vol. 140, pp. 1179-1188 (2014)
[Non-Patent Literature 10] Clinical Cancer Research, Vol. 23, No. 16, pp. 4651-4661 (2017)
[Non-Patent Literature 11] Future Oncology, Published Online:6 Apr 2018, https://doi.org/10.2217/fon-2017-0635 [Non-Patent Literature 12] Journal of Clinical Anesthesia, Vol. 26, pp. 106-117 (2014)

### SUMMARY OF THE INVENTION

The inventors found that postsurgical recurrence and/or metastasis of cancer is suppressed by administering a beta-blocker to a cancer patient during a perioperative period of cancer surgery.

Accordingly, the invention provides:
A short-acting beta-blocker for use in suppressing recurrence and/or metastasis of non-small cell lung cancer, wherein the short-acting beta-blocker is selected from the group consisting of landiolol, esmolol and a salt thereof and administered intravenously during a perioperative period of cancer surgery.

### BRIEF DESCRIPTION OF DRAWINGS

Fig. 1 shows Kaplan-Meier curves representing recurrence-free survival (RFS) of patients treated with landiolol hydrochloride during lung cancer surgery and patients not treated with it. The x-axis denotes number of months since the surgery and the y-axis denotes recurrence-free survival rate.
Fig. 2 shows Kaplan-Meier curves representing overall survival (OS) after lung cancer surgery. The x-axis denotes number of months since the surgery and the y-axis denotes event free rate (EFR).

### DETAILED DESCRIPTION

Unless otherwise defined, the terms used herein are read as generally understood by a skilled person in the technical fields such as organic chemistry, medicine, pharmacology, molecular biology, and microbiology. Definitions of several terms used herein are described below. The definitions herein take precedence over the general understanding.

When a numerical value is accompanied with the term "about", the value is intended to represent any value within the range of ± 10% of that value. For example, "about 20" means "a value between 18 and 22." A range defined with a value of the lower limit and a value of the upper limit covers all values between the both values of the limits, including the values of the both limits. When a range is accompanied with the term "about", the both limits are read as accompanied with the term. For example, "about 20 to 30" is read as "18 to 33."

In the invention, the short-acting beta-blocker is landiolol, esmolol or a salt thereof, preferably landiolol hydrochloride or esmolol hydrochloride, more preferably landiolol hydrochloride.

The salts as used herein are preferably pharmaceutically acceptable salts, especially pharmaceutically acceptable and water soluble salts. The pharmaceutically acceptable salts include alkali metal salts (e.g., potassium salts and sodium salts), alkaline earth metal salts (e.g., calcium salts and magnesium salts), ammonium salts, salts of pharmaceutically acceptable organic amines (e.g., tetramethyl ammonium, triethylamine, methylamine, dimethylamine, cyclopentylamine, benzylamine, phenethylamine, piperidine, monoethanolamine, diethanolamine, tris(hydroxymethyl)aminomethane, lysin, arginine, and N-methyl-D-glucamine), acid addition salts (e.g., salts of inorganic acids, such as hydrochlorides, hydrobromides, hydroiodides, sulfates, phosphates, and nitrates, as well as salts of organic acids, such as acetates, trifluoroacetates, lactates, tartrates, oxalates, fumarates, maleates, benzoates, citrates, methanesulfonates, ethanesulfonates, benzenesulfonates, toluenesulfonates, isethionates, glucoronates, and gluconates).

The beta-blockers mentioned above can be used safely with sufficiently low toxicity, because they have been approved and marketed as medicaments.

Short-acting beta-blockers are preferred in view of the hemodynamics of the patients during the cancer surgery. Intravenous or transdermal beta-blockers are also preferred because they can be administered together with an anesthetic drug.

The term "perioperative period" as used herein means a time period during which a cancer surgical procedure is performed, including preoperative, intraoperative, and postoperative periods. Specifically, the preoperative period begins before anesthetic induction prior to the cancer surgery (resection), e.g., immediately before the anesthetic induction, and the postoperative period includes several days until the condition of the patient after the cancer resection becomes stable, e.g., one to seven days from the end of the surgery. For example, the perioperative period begins preoperatively and ends at the termination of the anesthetic administration or later. Generally, any biological reaction due to an invasive procedure, e.g., increased blood pressure or tachycardia, does not occur in the preoperative period, as the period is prior to the surgery.

The administration period of the agent of the disclosure is preferably a period that begins before the anesthetic induction and ends seven days after the surgery, a period that begins before the anesthetic induction and ends six days after the surgery, a period that begins before the anesthetic induction and ends five days after the surgery, a period that begins before the anesthetic induction and ends four days after the surgery, or a period that begins before the anesthetic induction, for example, immediately before the anesthetic induction, and ends three days after the surgery. Alternatively, it is possible to predetermine a maximum administration period and stop the administration before the end of the period, for example one or two days before the end, depending on, for example, age, weight, gender, or clinical history of the patient, type, stage, progression, or metastatic potential of the cancer, operative procedure, organ subjected to the surgery, or combined therapy. For example, when an administration period is defined as ending within three days from the end of the surgery, the actual administration period may begin before the anesthetic induction and end optionally one, two or three days after the surgery. In this context, a period that begins before the anesthetic induction and ends one day after the surgery means a period that begins at the first administration of a beta-blocker prior to the anesthetic induction and ends about 24 hours after the first administration. Similarly, a period that ends two, three, four, five, six, or seven days after the surgery means a period that ends about 48, about 72, about 96, about 120, about 144, or about 168 hours after the first administration of a beta-blocker prior to the anesthetic induction, respectively.

The term "continuous administration" or "continuously administered" as used herein means that a beta-blocker is administered so that the blood concentration of the active ingredient is in the effective range over the period that is required for enabling the active ingredient to exhibit the effect to suppress postsurgical recurrence and/or metastasis of cancer. For example, intravenous continuous administration means administering an effective amount of a beta-blocker for a certain period, and may be achieved by administering a beta-blocker, e.g., in the form of an intravenous formulation or a lyophilized formulation dissolved in water for injection, via a pump for continuous injection, e.g., an infusion pump or a syringe pump.

The continuous administration may be performed intermittently with one or more appropriate washout periods as desired.

Whenever a beta-blocker is administered during a perioperative period, it can suppress postsurgical recurrence and/or metastasis of cancer. It is not necessary to administer a beta-blocker at other timing or via an oral route.

A beta-blocker may be administered at a dosage similar to the effective dosage known in the art. When it is continuously administered, the dosage may be adjusted within a certain range while the heart rate and blood pressure are monitored. For example, when landiolol hydrochloride is continuously administered parenterally, for example intravenously, the dosage may be about 0.1 to 10 µg/kg/min. Specific dosage of landiolol hydrochloride may be about 0.1 µg/kg/min, about 0.2 µg/kg/min, about 0.3 µg/kg/min, about 0.4 µg/kg/min, about 0.5 µg/kg/min, about 0.6 µg/kg/min, about 0.7 µg/kg/min, about 0.8 µg/kg/min, about 0.9 µg/kg/min, about 1 µg/kg/min, about 1.5 µg/kg/min, about 2 µg/kg/min, about 2.5 µg/kg/min, about 3 µg/kg/min, about 3.5 µg/kg/min, about 4 µg/kg/min, about 4.5 µg/kg/min, about 5 µg/kg/min, about 6 µg/kg/min, about 7 µg/kg/min, about 8 µg/kg/min, about 9 µg/kg/min, about 10 µg/kg/min, about 20 µg/kg/min, about 30 µg/kg/min, or about 40 µg/kg/min. A preferred dosage range herein may be determined by employing one dosage from the above-mentioned dosages as the lower limit and another dosage as the upper limit. Examples of such dosage range include about 0.2 to 8 µg/kg/min, about 0.3 to 5 µg/kg/min, about 0.4 to 3 µg/kg/min, about 0.5 to 2.5 µg/kg/min, about 0.2 to 20 µg/kg/min, about 0.3 to 15 µg/kg/min, about 0.4 to 10 µg/kg/min, and about 0.5 to 5 µg/kg/min.

The agent for suppressing recurrence and/or metastasis of cancer is administered to a mammal, for example a human, that is suffered from cancer and is in the perioperative period of cancer surgery (e.g., resection).

The term "agent for suppressing recurrence and/or metastasis of cancer" as used herein, which is also referred to as "antirecurrent and/or antimetastatic agent", means an agent capable of suppressing postsurgical recurrence and/or metastasis of cancer. The term "recurrence" of cancer means that cancer occurs again after the cancer was treated for example by surgery, including recurrence of primary cancer and further recurrence of recurrent cancer. The recurrence of cancer may be diagnosed by diagnostic imaging or biopsy analysis, or may be diagnosed when a symptom is aggravated. Organs where cancer recurs or is expected to recur include the organ where the cancer primarily occurred and any other organs. The term "metastasis" of cancer means recurrence of cancer in an organ other than the organ where the cancer primarily occurred. The term "suppressing recurrence and/or metastasis of cancer" includes preventing, reducing, or delaying recurrence and/or metastasis of cancer, or preventing, reducing, or delaying progression of cancer after recurrence and/or metastasis.

In the invention, the cancer is non-small cell lung cancer (e.g., squamous non-small cell lung cancer or non-squamous non-small cell lung cancer).

The effect of the agent of the disclosure, i.e., the effect to suppress recurrence and/or metastasis of cancer, may be enhanced by using the agent in combination with an additional therapy such as a known anticancer agent, cell-based therapy, and/or radiotherapy. Examples of the known anticancer agent include alkylating agents, antimetabolites, anticancerous antibiotics, plant alkaloids, antihormones, platinum compounds, cytokine preparations, molecular target agents, cancer immunotherapeutic agents, and cancer vaccines. Examples of the cell-based therapy include hematopoietic stem cell transplantation and chimeric antigen receptor (CAR) T-cell therapy. Examples of the radiotherapy include proton beam therapy, heavy ion radiotherapy, and boron neutron capture therapy (BNCT). The additional therapy may be applied at any time according to the standard therapeutic schedule of the therapy, without limited to a perioperative period. That is, the additional therapy may be applied before, during, or after the surgery.

The agent of the disclosure and at least one other anticancer agent may be administered via a single preparation containing the both agents or multiple separate preparations containing each agent individually. The separate preparations may be administered simultaneously or at different time points. When the separate preparations are administered at different time points, the preparation containing the agent of the disclosure may be administered first and then the preparation containing the other anticancer agent may be administered, or the preparation containing the other anticancer agent may be administered first and then the preparation containing the agent of the disclosure may be administered. The administration routes of each preparation may be same or different. Two or more other anticancer agents may be used in combination. The other anticancer agent may be a known anticancer agent, or an anticancer agent which will be found in future and works in a mechanism similar to that of a known anticancer agent.

Examples of the alkylating agent include nitrogen mustard N-oxide hydrochloride, cyclophosphamide, ifosfamide, melphalan, thiotepa, carboquone, busulfan, nimustine hydrochloride, dacarbazine, ranimustine, carmustine, chlorambucil, bendamustine, and mechlorethamine.

Examples of the antimetabolite include TS-1 (registered trademark), methotrexate, mercaptopurine, 6-mercaptopurine riboside, fluorouracil, tegafur, tegafururacil, carmofur, doxifluridine, cytarabine, enocitabine, tegafur-gimestat-otastat potassium, gemcitabine hydrochloride, cytarabine ocfosphate, procarbazine hydrochloride, and hydroxycarbamide.

Examples of the anticancerous antibiotic include actinomycin D, mitomycin C, daunorubicin hydrochloride, doxorubicin hydrochloride, aclarubicin hydrochloride, neocarzinostatin, pirarubicin hydrochloride, epirubicin (hydrochloride), idarubicin hydrochloride, chromomycin A3, bleomycin (hydrochloride), peplomycin sulfate, therarubicin, zinostatin stimalamer, and gemtuzumab ozogamicin.

Examples of the plant alkaloid include vinblastine sulfate, vincristine sulfate, vindesine sulfate, irinotecan hydrochloride, etoposide, flutamide, vinorelbine ditartrate, docetaxel hydrate, and paclitaxel.

Examples of the antihormone include estramustine phosphate sodium, mepitiostane, epitiostanol, goserelin acetate, fosfestrol (diethylstilbestrol phosphate), tamoxifen citrate, toremifene citrate, fadrozole hydrochloride hydrate, medroxyprogesterone acetate, bicalutamide, leuprorelin acetate, anastrozole, aminoglutethimide, androgen bicalutamide, and fulvestrant.

Examples of the platinum compound include carboplatin, cisplatin, nedaplatin, and oxaliplatin.

Examples of the cytokine preparation include IFN-alfa-2a, IFN-alfa-2b, PEG-IFN-alfa-2b, interferon beta natural, and interleukin-2.

Examples of the molecular target agent include rituximab, ibritumomab, ibritumomab tiuxetan, ocrelizumab, ofatuzumab, obinutuzumab, alemtuzumab, imatinib, sorafenib, sunitinib, bevacizumab, gefitinib, erlotinib, crizotinib, temsirolimus, everolimus, axitinib, pazopanib, regorafenib, cetuximab, ibrutinib, panitumumab, lapatinib, trastuzumab, and vandetanib.

Examples of the cancer immunotherapeutic agent include anti-PD-1 antibodies (e.g., human anti-human PD-1 monoclonal (neutralizing) antibodies, such as nivolumab and REGN-2810, and humanized anti-human PD-1 monoclonal (neutralizing) antibodies, such as pembrolizumab, PDR-001, BGB-A317, AMP-514 (also known as MEDI0680), ANB011 (also known as TSR-042), and STI-A1110), anti-PD-Ll antibodies (e.g., atezolizumab (also known as RG7446 or MPDL3280A), avelumab (also known as PF-06834635 or MSB0010718C), durvalumab (also known as MEDI4736), BMS-936559, STI-1010, STI-1011, and STI-1014), PD-1 antagonists (e.g., AUNP-12), anti-PD-L2 antibodies, PD-L1 fusion proteins, PD-L2 fusion proteins (e.g., AMP-224), anti-CTLA-4 antibodies (e.g., ipilimumab and tremelimumab), anti-LAG-3 antibodies (e.g., BMS-986016 and LAG525), anti-Tim3 antibodies (e.g., MBG453), anti-KIR antibodies (e.g., lirilumab), anti-BTLA antibodies, anti-TIGIT antibodies, anti-VISTA antibodies, anti-CD137 antibodies (e.g., urelumab), anti-OX40 antibodies (e.g., MEDI6469), anti-HVEM antibodies, anti-CD27 antibodies (e.g., varlilumab), anti-GITR antibodies (e.g., MK-4166 and TRX-518), anti-CD28 antibodies, anti-CCR4 antibodies (e.g., mogamulizumab), anti-CD4 antibodies (e.g., MTRX-1011A, TRX-1, ibalizumab, huB-F5, zanolimumab, 4162W94, clenoliximab, keliximab, AD-519, PRO-542, cedelizumab, TNX-355, dacetuzumab, tregalizumab, priliximab, MDX-CD4, CAMPATH-9, and IT1208), TLR agonists, and STING agonists (e.g., MIW815).

Examples of the cancer vaccine include tumor antigen peptide vaccines and dendritic cell vaccines.

The following examples do not restrict or limit the invention and are for illustrative purposes only.

### EXAMPLES

### Example 1: Analysis of effects of landiolol hydrochloride in patients who underwent lung resection surgery

### 1. Study type and design

Ethics committee approval was obtained before the study was started. Among the patients who underwent lung lobectomy, the outcomes were compared between those who were treated with landiolol hydrochloride and those who were not treated with it.

### 2. Methods

### 1) Data collection

For each patient the following information was drawn from the medical chart from May 2012 to March 31, 2017 and recorded in clinical report form (CRF).
- Background of the patient (date of birth, sex, stage of lung cancer, weight, height, and performance status)
- Date of the lung cancer surgery
- Whether landiolol hydrochloride was administered or not, and if administered, the dose thereof
- Date on which recurrence was diagnosed (date of diagnostic imaging or date on which recurrence was clinically diagnosed)
- Date on which recurrence-free survival was last confirmed
- Date of death
- Date on which survival was last confirmed
- Whether adjuvant chemotherapy was applied or not

### 2) Endpoints

### 2)-1 Primary endpoint

The 2-year recurrence-free survival rate was compared between the patients who had been treated with landiolol hydrochloride and the patients who had not been treated with it to evaluate the effect of landiolol hydrochloride.

### 2)-1-1 Method for assessing primary endpoint

Kaplan-Meier method as described below was employed to calculate the 2-year recurrence-free survival rate.

The recurrence was defined as follows;
- The recurrence included both of recurrence diagnosed on the basis of diagnostic imaging and clinical recurrence diagnosed on the basis of aggravation of a symptom instead of the diagnostic imaging. The date of recurrence was the date of the diagnostic imaging or the date on which the clinical recurrence was diagnosed. Even if an increased tumor marker was detected previously, the fact alone was not regarded as indicating recurrence and the date of recurrence was the date of the diagnostic imaging or the date on which the clinical recurrence was diagnosed. When no recurrence was diagnosed during the observation period, the patients were censored on the day of final confirmation of recurrence-free survival.
- When recurrence was diagnosed by the diagnostic imaging, the event was regarded as having occurred on the date of the diagnostic imaging that gave the definitive diagnosis, not the date of the diagnostic imaging that raised the suspicion of recurrence. When recurrence was clinically diagnosed, the event was regarded as having occurred on the date of the diagnosis.
- Definitive diagnosis of recurrence may be based on biopsy analysis. When recurrence was successfully diagnosed by the clinical diagnosis before the biopsy analysis, the event was regarded as having occurred on the date of the clinical diagnosis. When recurrence was not successfully diagnosed by the clinical diagnosis alone, but finally diagnosed by the biopsy analysis, the event was regarded as having occurred on the date of the biopsy analysis.
- Occurrence of any one of secondary cancer, metachronous double cancers, and metachronous multiple cancers was not regarded as the event or the censor. Unless another event was observed, the patient was regarded as being recurrence-free.

### 2)-2 Secondary endpoints

The recurrence-free survival and the overall survival were compared between the patients who had been treated with landiolol hydrochloride and the patients who had not been treated with it to evaluate the effects of landiolol hydrochloride.

### 2)-2-1 Method for assessing secondary endpoints

The recurrence-free survival and the overall survival were calculated according to the following equations;

The recurrence-free survival (days) = "date of diagnosis of recurrence or date of death for any cause, whichever came earlier" - "date of surgery" + 1

The overall survival (days) = "date of death for any cause" - "date of surgery" + 1

The term "date of diagnosis of recurrence or date of death for any cause, whichever came earlier" is defined as below.
- The recurrence included both of recurrence diagnosed on the basis of diagnostic imaging and clinical recurrence diagnosed on the basis of aggravation of a symptom instead of the diagnostic imaging. The date of recurrence was the date of the diagnostic imaging or the date on which the clinical recurrence was diagnosed. Even if an increased tumor marker was detected previously, the fact alone was not regarded as indicating recurrence and the date of recurrence was the date of the diagnostic imaging or the date on which clinical recurrence was diagnosed. When no recurrence was diagnosed during the observation period, the patients were censored on the day of final confirmation of recurrence-free survival.
- When recurrence was diagnosed by the diagnostic imaging, the event was regarded as having occurred on the date of the diagnostic imaging that gave the definitive diagnosis, not the date of the diagnostic imaging that raised the suspicion of recurrence. When recurrence was clinically diagnosed, the event was regarded as having occurred on the date of the diagnosis.
- Definitive diagnosis of recurrence may be based on biopsy analysis. When recurrence was successfully diagnosed by the clinical diagnosis before the biopsy analysis, the event was regarded as having occurred on the date of the clinical diagnosis. When recurrence was not successfully diagnosed by the clinical diagnosis alone, but finally diagnosed by the biopsy analysis, the event was regarded as having occurred on the date of the biopsy analysis.
- Occurrence of any one of secondary cancer, metachronous double cancers, and metachronous multiple cancers was not regarded as the event or the censor. Unless another event was observed, the patient was regarded as being recurrence-free.

### 2)-3 Statistical analysis

### 2)-3-1 Statistical analysis of primary endpoint

(1) Frequency of the events and the censors was calculated for each group. Frequency of the details about the events and the censors was calculated for each group.
(2) The recurrence-free survival rate over first two postoperative years was calculated for each group by Kaplan-Meier method, and the two-sided 95% confidence interval was calculated.
(3) Group-wise comparison was performed by Chi-square test. Difference of the 2-year recurrence-free survival rate between the patients who had been treated with landiolol hydrochloride and the patients who had not been treated with it and the two-sided 95% confidence interval of the difference were calculated.

### 2)-3-2 Statistical analysis of secondary endpoints

(1) Kaplan-Meier curves of each endpoint were generated for each group. The median and the two-sided 95% confidence interval thereof were calculated for each group by Kaplan-Meier method.
(2) Group-wise comparison was performed by log-rank test.
(3) Using Cox proportional hazard model in which the landiolol hydrochloride administration is a single factor, Hazard Ratio of the administration group to the placebo group and the two-sided 95% confidence interval thereof were calculated.

### 3. Selection of subjects

The patients who fulfilled all the following inclusion and exclusion criteria were included in the study.

### 3-1: Inclusion criteria

(1) Men and women aged under 85
(2) ASA1-2
(3) Patients who underwent lung resection surgery for malignant lung tumor

### 3-2: Exclusion criteria

(1) Patients with supraventricular arrhythmia (atrial fibrillation or atrial flutter)
(2) Patients with sick sinus syndrome, including those having an implanted pacemaker
(3) Patients with severe heart failure (NYHA functional class III or higher)
(4) Patients with atrioventricular block of second-degree or higher
(5) Patients with moderate to severe valvular heart disease
(6) Patients who underwent acute cardiovascular event, cerebrovascular event, or infectious inflammation within 60 days
(7) Patients having tendency of bradycardia (resting heart rate was below 55 bpm)
(8) Patients having systolic blood pressure less than 80 mmHg
(9) Patients who were treated with a beta-blocker prior to the surgery
(10) Patients with severe bronchial asthma
(11) Patients having typical honeycomb lung due to obstructive pulmonary disease (COPD grade II or more severe) or a diffuse lung disease (IP)
(12) Patients with atrial fibrillation due to an identified cause, for example, electrolyte abnormality, WPW syndrome, or hyperthyroidism
(13) Patients who were treated with an antiarrhythmic drug other than digitalis prior to the surgery
(14) Patients for whom beta-blockers were contraindicated
(15) Patients with a chronic inflammatory disease
(16) Patients who underwent a severe hepatic disorder
(17) Patients who underwent a severe renal disorder
(18) Patients whom the principal investigator found inadequate for the study due to any reason

### 4. Results

### 4-1: Backgrounds of patients

Backgrounds of the patients obtained as described in item 1) of 2. Methods above are summarized in Table 1. Landiolol hydrochloride was administered to the patients in the test group by intravenous continuous administration at the dosage of 2.5 µg/kg/min over the period from the induction to completion of anesthesia. Saline was continuously administered to the patients in the control group (placebo group).

**[Table 1]**

| | Test group N = 28 n (%) | Control group N = 29 n (%) | Sum N = 57 n (%) |
|---|---|---|---|
| Age | | | |
| Mean (SD) | 70.3 (7.5) | 69.1 (8.4) | 69.7 (7.9) |
| Median | 69.5 | 70.0 | 70.0 |
| Min-Max | 50-84 | 52-82 | 50-84 |

| Sex | | | |
|---|---|---|---|
| Male | 15 (53.6) | 17 (58.6) | 32 (56.1) |
| Female | 13 (46.4) | 12 (41.4) | 25 (43.9) |

| Pathological stage | | | |
|---|---|---|---|
| IA | 16 (57.1) | 14 (48.3) | 30 (52.6) |
| IB | 3 (10.7) | 7 (24.1) | 10 (17.5) |
| IIA | 5 (17.9) | 4 (13.8) | 9 (15.8) |
| IIB | 1 (3.6) | 3 (10.3) | 4 (7.0) |
| IIIA | 2 (7.1) | 1 (3.4) | 3 (5.3) |
| IIIB | 1 (3.6) | 0 (0.0) | 1 (1.8) |
| | | | |

| Body weight (kg) | | | |
|---|---|---|---|
| Mean (SD) | 57.2 (8.4) | 54.5 (10.1) | 55.8 (9.3) |
| Median | 56.3 | 51.6 | 55.0 |
| Min - Max | 38.0-71.0 | 36.8-74.0 | 36.8-74.0 |
| | | | |

| BMI (kg/m^2) | | | |
|---|---|---|---|
| Mean (SD) | 22.5 (2.6) | 21.3 (3.0) | 21.9 (2.8) |
| Median | 22.4 | 21.0 | 21.3 |
| Min-Max | 16.9-27.3 | 14.4-28.0 | 14.4-28.0 |
| | | | |

| Adjuvant chemotherapy | | | |
|---|---|---|---|
| Yes | 6 (21.4) | 2 (6.9) | 8 (14.0) |
| No | 22 (78.6) | 27 (93.1) | 49 (86.0) |

### 4-2: Primary endpoint

The primary endpoint, the 2-year recurrence-free survival rate, was compared between the test group and the control group as described in items 2)-1 and 2)-1-1 of 2. Methods above. The results are shown in Table 2. The 2-year recurrence-free survival rates were 89.3% and 75.9 % in the test group and the control group, respectively.

**[Table 2]**

| | Test group N = 28 n (%) | Control group N = 29 n (%) |
|---|---|---|
| Event | 3 (10.7) | 7 (24.1) |
| Censor | 25 (89.3) | 22 (75.9) |
| | | |
| 2-year Recurrence-free survival rate | 89.3 | 75.9 |
| [95% CI] | [77.8, 100.7] | [60.3, 91.4] |
| | | |
| Chi-square test ^{a)} | p=0.1828 | |

| | | |
|---|---|---|
| The periods are shown in months, wherein one month is equal to 30.4375 days. a)* : p<0.05, N.S. : p>=0.05 | | |

### 4-3: Secondary endpoints

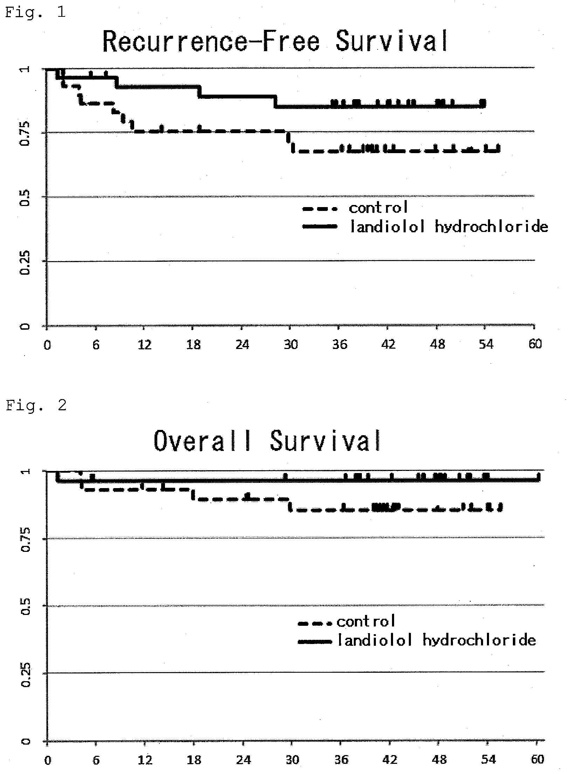

Figs. 1 and 2 show the recurrence-free survival and the overall survival, respectively, which were compared between the test group and the control group as described in items 2)-2 and 2)-2-1 of 2. Methods. In the both figures, the upper curves indicate the results of the test group with the administration of landiolol hydrochloride and the lower curves indicate the results of the control group without the administration.

There was a tendency that the administration of landiolol hydrochloride prolongs both of the recurrence-free survival and the overall survival.

These results indicate that administration of landiolol hydrochloride during a perioperative period of lung cancer surgery can suppress recurrence and/or metastasis of the cancer.

### Example 2: Effects of landiolol hydrochloride administered during a perioperative period to suppress postsurgical recurrence of non-small cell lung cancer

Patients with non-small cell lung cancer who will undergo surgery for complete resection of the cancer are involved in the study. For evaluating effectiveness and safety of landiolol hydrochloride in the patients, a multicenter, unblinded, randomized, controlled, investigator-initiated clinical trial is carried out as described below. The control group includes the patients who undergo the surgery but are not treated with landiolol hydrochloride.

Primary endpoints: recurrence-free survival (RFS) over first two postoperative years and overall survival (OS)

Secondary endpoints: <Effectiveness> For example, whether the patients are subjected to treatment of recurrence or not, and if subjected, the period until the treatment; the time until OS or the treatment of recurrence, <Safety> adverse events, incidence of postoperative complication, blood pressure, 12-lead electrocardiogram, and general laboratory test
Dosage: 2.5 µg/kg/min at the beginning and adjusted in the range of 0.5 to 5 µg/kg/min
Administration period: continuous intravenous administration is started immediately before the anesthetic induction and kept for 72 hours

The study confirms the effect of landiolol hydrochloride administered during a perioperative period of lung cancer surgery to suppress recurrence and/or metastasis of the cancer.

### INDUSTRIAL APPLICABILITY

Recurrence and/or metastasis of cancer can be suppressed by administering a beta-blocker, preferably landiolol hydrochloride, to a cancer patient during a perioperative period of cancer surgery. This desired effect can be achieved with less burden on the patient because the beta-blocker is administered for the short perioperative period and no chronic administration is necessary.

## Claims

1. A short-acting beta-blocker for use in suppressing recurrence and/or metastasis of non-small cell lung cancer, wherein the short-acting beta-blocker is selected from the group consisting of landiolol, esmolol, and a salt thereof and administered intravenously during a perioperative period of cancer surgery.

2. The short-acting beta-blocker for use according to claim 1, wherein the short-acting beta-blocker is continuously administered over the period that begins preoperatively and ends at the termination of the anesthetic administration or later.

3. The short-acting beta-blocker for use according to claim 1 or 2, wherein the short-acting beta-blocker is continuously administered over the period that begins preoperatively and ends within about 168 hours from the end of the surgery.

4. The short-acting beta-blocker for use according to any one of claims 1 to 3, wherein the short-acting beta-blocker is continuously administered over the period that begins preoperatively and ends within about 72 hours from the end of the surgery.

5. The short-acting beta-blocker for use according to any one of claims 1 to 4, wherein the short-acting beta-blocker is selected from the group consisting of landiolol and a salt thereof.

6. The short-acting beta-blocker for use according to any one of claims 1 to 5, wherein the short-acting beta-blocker is landiolol hydrochloride.

7. The short-acting beta-blocker for use according to claim 6, wherein the dosage of landiolol hydrochloride is about 0.1 to 10 µg/kg/min.

8. The short-acting beta-blocker for use according to claim 6 or 7, wherein the dosage of landiolol hydrochloride is about 0.5 to 5 µg/kg/min.

9. The short-acting beta-blocker for use according to any one of claims 6 to 8, wherein the dosage of landiolol hydrochloride is about 0.5 to 2.5 µg/kg/min.

10. The short-acting beta-blocker for use according to any one of claims 6 to 9, wherein the dosage of landiolol hydrochloride is about 2.5 µg/kg/min.

11. The short-acting beta-blocker for use according to any one of claims 1 to 4, wherein the short-acting beta-blocker is selected from the group consisting of esmolol and a salt thereof.

## Patentansprüche

1. Kurzwirkender Betablocker zur Verwendung beim Unterdrücken eines Rezidivs und/oder einer Metastase von nicht-kleinzelligem Lungenkrebs, wobei der kurzwirkende Betablocker ausgewählt ist aus der Gruppe bestehend aus Landiolol, Esmolol und einem Salz davon und intravenös während eines perioperativen Zeitraums einer Krebsoperation verabreicht wird.

2. Kurzwirkender Betablocker zur Verwendung nach Anspruch 1, wobei der kurzwirkende Betablocker kontinuierlich über einen Zeitraum verabreicht wird, der präoperativ beginnt und mit der Beendigung der Verabreichung des Anästhetikums oder später endet.

3. Kurzwirkender Betablocker zur Verwendung nach Anspruch 1 oder 2, wobei der kurzwirkende Betablocker kontinuierlich über den Zeitraum verabreicht wird, der präoperativ beginnt und innerhalb etwa 168 Stunden von dem Ende der Operation endet.

4. Kurzwirkender Betablocker zur Verwendung nach einem der Ansprüche 1 bis 3, wobei der kurzwirkende Betablocker kontinuierlich über den Zeitraum verabreicht wird, der präoperativ beginnt und innerhalb etwa 72 Stunden von dem Ende der Operation endet.

5. Kurzwirkender Betablocker zur Verwendung nach einem der Ansprüche 1 bis 4, wobei der kurzwirkende Betablocker ausgewählt ist aus der Gruppe bestehend aus Landiolol und einem Salz davon.

6. Kurzwirkender Betablocker zur Verwendung nach einem der Ansprüche 1 bis 5, wobei der kurzwirkende Betablocker Landiololhydrochlorid ist.

7. Kurzwirkender Betablocker zur Verwendung nach Anspruch 6, wobei die Dosierung von Landiololhydrochlorid etwa 0,1 bis 10 µg/kg/min beträgt.

8. Kurzwirkender Betablocker zur Verwendung nach Anspruch 6 oder 7, wobei die Dosierung von Landiololhydrochlorid etwa 0,5 bis 5 µg/kg/min beträgt.

9. Kurzwirkender Betablocker zur Verwendung nach einem der Ansprüche 6 bis 8, wobei die Dosierung von Landiololhydrochlorid etwa 0,5 bis 2,5 µg/kg/min beträgt.

10. Kurzwirkender Betablocker zur Verwendung nach einem der Ansprüche 6 bis 9, wobei die Dosierung von Landiololhydrochlorid etwa 2,5 µg/kg/min beträgt.

11. Kurzwirkender Betablocker zur Verwendung nach einem der Ansprüche 1 bis 4, wobei der kurzwirkende Betablocker ausgewählt ist aus der Gruppe bestehend aus Esmolol und einem Salz davon.

## Revendications

1. Bêta-bloquant de courte durée d'action pour l'utilisation dans la suppression de récurrence et/ou de métastase de cancer du poumon non à petites cellules, dans lequel le bêta-bloquant de courte durée d'action est sélectionné parmi le groupe constitué de landiolol, d'esmolol, et d'un sel de ceux-ci et administré de façon intraveineuse durant une période périopératoire de chirurgie de cancer.

2. Bêta-bloquant de courte durée d'action pour l'utilisation selon la revendication 1, dans lequel le bêta-bloquant de courte durée d'action est administré en continu durant la période qui commence de façon préopératoire et se termine à la fin de l'administration d'anesthésique ou après.

3. Bêta-bloquant de courte durée d'action pour l'utilisation selon la revendication 1 ou 2, dans lequel le bêta-bloquant de courte durée d'action est administré en continu durant la période qui commence de façon préopératoire et se termine au sein d'environ 168 heures après la fin de la chirurgie.

4. Bêta-bloquant de courte durée d'action pour l'utilisation selon l'une quelconque des revendications 1 à 3, dans lequel le bêta-bloquant de courte durée d'action est administré en continu durant la période qui commence de façon préopératoire et se termine au sein d'environ 72 heures après la fin de la chirurgie.

5. Bêta-bloquant de courte durée d'action pour l'utilisation selon l'une quelconque des revendications 1 à 4, dans lequel le bêta-bloquant de courte durée d'action est sélectionné parmi le groupe constitué de landiolol et d'un sel de celui-ci.

6. Bêta-bloquant de courte durée d'action pour l'utilisation selon l'une quelconque des revendications 1 à 5, dans lequel le bêta-bloquant de courte durée d'action est du chlorhydrate de landiolol.

7. Bêta-bloquant de courte durée d'action pour l'utilisation selon la revendication 6, dans lequel le dosage de chlorhydrate de landiolol est d'environ 0,1 à 10 µg/kg/min.

8. Bêta-bloquant de courte durée d'action pour l'utilisation selon la revendication 6 ou 7, dans lequel le dosage de chlorhydrate de landiolol est d'environ 0,5 à 5 µg/kg/min.

9. Bêta-bloquant de courte durée d'action pour l'utilisation selon l'une quelconque des revendications 6 à 8, dans lequel le dosage de chlorhydrate de landiolol est d'environ 0,5 à 2,5 µg/kg/min.

10. Bêta-bloquant de courte durée d'action pour l'utilisation selon l'une quelconque des revendications 6 à 9, dans lequel le dosage de chlorhydrate de landiolol est d'environ 2,5 µg/kg/min.

11. Bêta-bloquant de courte durée d'action pour l'utilisation selon l'une quelconque des revendications 1 à 4, dans lequel le bêta-bloquant de courte durée d'action est sélectionné parmi le groupe constitué d'esmolol et d'un sel de celui-ci.
